# EUROPEAN PATENT APPLICATION

(11) **EP 4 129 386 A1**
(43) Date of publication of application: **08.02.2023**
(21) Application number: 21778806.6
(22) Date of filing: 31.03.2021
(51) Int. Cl.: A61M 25/10, A61B 18/04, A61B 6/00, A61B 6/12

(54) **IMAGE ANALYSIS DEVICE, METHOD FOR CONTROLLING IMAGE ANALYSIS DEVICE, IMAGE ANALYSIS SYSTEM, AND METHOD FOR CONTROLLING IMAGE ANALYSIS SYSTEM**

(30) Priority: 31.03.2020 JP 2020064935
(71) Applicant: Toray Industries, Inc., Tokyo 103-8666 (JP)
(72) Inventor: KIMURA, Shuya, Otsu-shi, Shiga 520-8558 (JP)
(74) Representative: Kador & Partner PartG mbB
(86) International application number: PCT/JP2021/013782
(87) International publication number: WO 2021/201080

(57) **Abstract**

The present invention provides an image analysis device capable of outputting evaluation information for use in the balloon catheter treat ment for subjects, in real time during a procedure using a balloon cathet er. The image analysis device comprises: an input unit configured to inp ut captured image data of a two-dimensional captured image of a balloo n catheter inserted into an organ of the subject and pressed against an i nner surface of the organ; a processing unit configured to obtain evalua tion image data of the two-dimensional evaluation image, by executing a n image processing on the captured image based on the captured image data input by the input unit, and configured to obtain evaluation informa tion regarding a state of the balloon catheter pressed against the inner s urface of the organ based on the evaluation image data; and an output unit configured to output the evaluation information acquired by the proc essing unit.

## Description

### [Technical Field]

The present invention relates to an image analysis device, a contr ol method for an image analysis device, an image analysis system, and a control method for an image analysis system.

### [Background Art]

Conventionally, by inserting a catheter into an organ such as a blood vessel, an examination or treatment is performed on a lesion.

For example, a support system has been proposed in which the contact state between the catheter and the myocardium is investigated in advance to convert it into data, and the result is displayed on the screen while performing the procedure (see, for example, Patent Documents 1 and 2).

Furthermore, a system for determining and displaying the catheter position during ablation in real time has been proposed (see, for example, Patent Document 3).

### [Prior Art Documents]

### [Patent Literature]

[Patent Document 1] JP2017-148142
[Patent Document 2] JP2017-94073
[Patent Document 3] JP2019-103809

### [Disclosure of the Invention]

### [Problem to be Solved by the Invention]

Here, ablation with a balloon catheter in balloon catheter treatment is a procedure in which heat is transferred to the entire contact portion by ablation while contacting and pressing the balloon against the myocardium to treat multiple points at once. For example, the Satake Hot Balloon (Registered Trademark) applied to this balloon catheter treatment is to control the temperature by a coil electrode in the balloon and a thermocouple welded to the portion thereof.

The inventor of this application has confirmed that, in such a Satake Hot Balloon (Registered Trademark), if the positional relationship of the balloon operation part (the sheath end and the tip tube) changes during the ablation, the surface temperature of the balloon will change. In addition, in this Satake Hot Balloon (Registered Trademark), a device that makes it easier to determine during the procedure whether the temperature of the balloon is correctly transmitted to the myocardium is requested by the doctor.

Thus, the evaluation of the coaxiality, between the end of the sheath and the tip tube, which indicates that the controlled temperature of the balloon and the surface temperature of the actual balloon are in the same state, and the evaluation of the shape, which indicates that the balloon is in contact with the ablation point, are important for an effective procedure using a balloon catheter.

Therefore, in the present invention, the coaxiality of the catheter is evaluated, by performing image processing on the fluoroscopic image used by the doctor during the procedure, from the detection of the end of the sheath and the tip tube. Then, the size of the balloon before being pressed against the myocardium is estimated from the number of pixels of the balloon detected on the fluoroscopic image. The amount of balloon deformation before and after the procedure is quantified by comparing it with the balloon size calculated from the input image. An image analysis device has been developed that can output an index during ablation treatment by processing and displaying this information in real time.

As described above, an object of the present invention is to provide an image analysis device capable of outputting evaluation information for use in the balloon catheter treatment for subjects (e.g., humans), in real time during a procedure using a balloon catheter.

### [Solution to Problem]

The image analysis device according to the embodiment according to one aspect of the present invention includes: an input unit configured to input captured image data of a two-dimensional captured image of a balloon catheter inserted into an organ of the subject and pressed against an inner surface of the organ; a processing unit configured to obtain evaluation image data of a two-dimensional evaluation image, by executing an image processing on the captured image based on the captured image data input by the input unit, and configured to obtain evaluation information regarding a state of the balloon catheter pressed against the inner surface of the organ based on the evaluation image data; and an output unit configured to output the evaluation information acquired by the processing unit.

In the image processing, the processing unit acquires the evaluati on information including the measured value obtained by measuring the shape of the balloon catheter included in the captured image or the inde x calculated based on the measured value, and the output unit outputs t he evaluation information acquired by the processing unit.

In the image analysis device, wherein the output unit is a display device that displays the evaluation information to the outside.

The captured image is a two-dimensional X-ray fluoroscopic image taken by seeing through the balloon catheter located in the organ of th e subject, using X-rays.

In the image analysis device, wherein the balloon catheter compri ses: a sheath; a catheter tube housed in the sheath; a balloon having o ne end connected to an end of the sheath; and a tip tube connected to the other end of the balloon and configured to lead the sheath.

In the image processing, the processing unit detects the balloon I ocated in the organ of the subject, the end of the sheath in contact with one end of the balloon or the end of the catheter tube, and tip tube in contact with the other end of the balloon, from the captured image, by e xecuting noise reduction processing, shading detection processing, or patt ern matching processing on the captured image input by the input unit.

In the image processing, the processing unit detects the balloon f rom the captured image by executing the Sobel filter processing or the R ing filter processing on the captured image.

In the image processing, the processing unit detects the end porti on of the sheath and the tip tube by a rectangular detection filter from a vicinity of the edge of the balloon detected from the captured image.

In the image processing, a size of the first rectangular detection f ilter for detecting the end of the sheath is different from a size of the se cond rectangular detection filter for detecting the tip tube, and the size o f the first rectangular detection filter and the size of the second rectangu lar detection filter are set to sizes that do not interfere with a wire.

In the image processing, the processing unit detects the coaxialit y indicating a relationship between a first axial direction, of the end of t he sheath or the catheter tube, and a second axial direction, of the tip t ube, from the image-processed captured image, and the processing unit acquires the detected coaxiality as the evaluation information.

In the image processing, the processing unit calculates a first ang le in the first axial direction of the end of the sheath with respect to the reference direction and a second angle in the second axial direction of t he tip tube with respect to the reference direction, in the two-dimension al captured image, and the processing unit obtains an angle difference, which is the difference between the first angle and the second angle, as the evaluation information of the coaxiality.

In the image processing, the processing unit obtains number of fi rst pixels, which is total number of pixels of the captured image correspo nding to a detected first shape of the balloon, and obtains number of se cond pixels, which is total number of pixels of the captured image corres ponding to a theoretical second shape of the balloon before being presse d against the inner surface of the organ, and the processing unit evaluat es the shape of the balloon pressed against the inner surface of the orga n based on a difference between the number of first pixels and the num ber of second pixels.

In the image processing, the processing unit detects a first contac t point between the detected edge of the balloon and the end of the she ath, and a second contact point between the detected edge of the balloo n and the tip tube, the processing unit calculates a length of the line se gment connecting the first contact point and the second contact point de tected, as a diameter of the balloon before being pressed against the inn er surface of the organ, the processing unit calculates a first balloon are a of the first shape of the balloon whose edge is detected in a pressed s tate pressed against the inner surface of the organ, the processing unit obtains a theoretical second shape of a circle or ellipse of the balloon be fore the balloon is pressed against the inner surface of the organ, based on the calculated diameter of the balloon and the first balloon area, the processing unit divides the first shape of the balloon in the pressed state into a plurality of regions, the processing unit calculates area of each of the regions of the divided first shape, the processing unit divides the th eoretical second shape of the balloon into a plurality of regions in the sa me manner as the dividing of the first shape, the processing unit calcula tes the area of each region of the second shape divided, and the process ing unit calculates a rate of change in the shape of the balloon due to p ressing against the inner surface of the organ, by comparing the area of each region of the first shape of the balloon and the area of each region of the second shape of the balloon for each region corresponding to a p osition in the captured image.

In the image processing, the processing unit divides the first shap e of the balloon in the pressed state into the plurality of regions along t he line segment, and the processing unit divides the theoretical second s hape of the balloon into the plurality of regions along the line segment.

In the image processing, the processing unit divides the first shap e of the balloon in the pressed state into a plurality of regions around th e line segment, and divides the theoretical second shape of the balloon i nto a plurality of regions around the line segment.

In the image processing, the processing unit divides the first shap e of the balloon in the pressed state into six regions, by dividing along t he line segment into sections every 1/3 of the length of the line segmen t and dividing by the center of the line segment, and the processing unit divides the theoretical second shape of the balloon into six regions, by dividing along the line segment into sections every 1/3 of the length of t he line segment and dividing by the center of the line segment.

The image analysis device further includes a storage unit storing the evaluation information.

In the image processing, the processing unit evaluates the change in the coaxiality of the balloon catheter within a predetermined period during the balloon catheter treatment, by performing the analysis of the moving image based on the captured image input from the input unit during the balloon catheter treatment.

In image processing, the processing unit evaluates the change in the shape of the balloon of the balloon catheter within a predetermined period during the treatment of the balloon catheter, by performing the analysis of the moving image based on the captured image input from the input unit during the balloon catheter treatment.

A control method, for an image analysis device, according to the embodiment according to one aspect of the present invention, the image analysis device comprises: an input unit configured to input captured image data of a two-dimensional captured image obtained by the image pickup device; a processing unit; and output unit, wherein the processing unit obtains evaluation image data of a two-dimensional evaluation image, by executing an image processing on the captured image based on the captured image data input by the input unit, and obtains evaluation information regarding a state of the balloon catheter pressed against the inner surface of the organ based on the evaluation image data, and wherein the output unit outputs the evaluation information acquired by the processing unit.

An image analysis system, according to the embodiment accordin g to one aspect of the present invention, that outputs evaluation informa tion for use in balloon catheter treatment for subjects, the image analysi s system comprises: an image pickup device configured to obtain capture d image data of a two-dimensional captured image, by imaging a balloon catheter inserted into the organ of the subject and pressed against the inner surface of the organ; and an image analysis device configured to o utput evaluation information for use in balloon catheter treatment for the subject based on the captured image data of the captured image, wher ein the image analysis device comprises: an input unit configured to inpu t captured image data of a two-dimensional captured image obtained by the image pickup device; a processing unit configured to obtain evaluati on image data of a two-dimensional evaluation image, by executing an i mage processing on the captured image based on the captured image da ta input by the input unit, and configured to obtain evaluation informatio n regarding a state of the balloon catheter pressed against the inner surf ace of the organ based on the evaluation image data; and an output uni t configured to output the evaluation information acquired by the process ing unit.

A control method, for an image analysis system, according to the embodiment according to one aspect of the present invention, the image analysis system outputs evaluation information for use in balloon cathete r treatment for subjects, the image analysis system comprises: an image pickup device configured to obtain captured image data of a two-dimens ional captured image, by imaging a balloon catheter inserted into the org an of the subject and pressed against the inner surface of the organ; an d an image analysis device configured to output evaluation information fo r use in balloon catheter treatment for the subject based on the capture d image data of the captured image, wherein the image analysis device comprises: an input unit configured to input captured image data of a tw o-dimensional captured image obtained by the image pickup device; a pr ocessing unit; and output unit, wherein the processing unit obtains evalu ation image data of a two-dimensional evaluation image, by executing an image processing on the captured image based on the captured image data input by the input unit, and obtains evaluation information regardin g a state of the balloon catheter pressed against the inner surface of the organ based on the evaluation image data, and wherein the output unit outputs the evaluation information acquired by the processing unit.

### [Effects of the Invention]

An image analysis device according to one aspect of the present i nvention can output evaluation information for use in balloon catheter tre atment for subjects in real time during a procedure using a balloon cath eter.

### [Brief Description of the Drawings]

[FIG. 1] FIG. 1 is a diagram showing an example of a configuration inclu ding an image analysis system comprising an image analysis device 100 according to an embodiment and a balloon catheter system 10 applied to balloon catheter treatment.
[FIG. 2A] FIG. 2A is a diagram showing an example of a configuration in the vicinity of a balloon of a balloon catheter in an inflated state.
[FIG. 2B] FIG. 2B is a diagram showing an example of a configuration in the vicinity of a balloon of a balloon catheter in a deflated state.
[FIG. 3] FIG. 3 is a diagram showing an example of the configuration of the image analysis device 100 shown in FIG.1.
[FIG. 4] FIG. 4 is a diagram showing an example of a control method of the image analysis device 100 shown in FIG.3.
[FIG. 5A] FIG. 5A is a diagram showing an example of image processing that executes a Sobel filter process and a Ring filter process for detecting a balloon in a captured image.
[FIG. 5B] FIG. 5B is a diagram showing an example of image processing for executing a process of detecting the edge of a balloon by performing a shading calculation on radiation from the center point of the balloon detected by the process of FIG. 5A.
[FIG. 5C] FIG. 5C is a diagram showing an example of image processing for executing a rectangular detection filter process for detecting an end portion and a tip tube of a sheath in a captured image.
[FIG. 5D] FIG. 5D is a diagram showing an example of image processing for calculating the angle difference between the end portion of the sheath and the tip tube in a captured image.
[FIG. 6] FIG. 6 is a diagram showing an example of a model of rectangular detection filtering processing shown in FIG. 5C.
[FIG. 7] FIG. 7 is a diagram showing a detailed example of a step of evaluating a shape in the control flow shown in FIG. 4.
[FIG. 8A] FIG. 8A is a diagram showing an example of a two-dimensional image of a balloon catheter in a state of being pressed against the inner surface (the myocardium) of an organ of a subject, which is acquired in image processing.
[FIG. 8B] FIG. 8B is a diagram showing an example in which a two-dimensional image of the balloon shown in FIG. 8A and the theoretical outer shape of the balloon before pressing are superimposed.
[FIG. 8C] FIG. 8C is a diagram showing an example of a model in which the theoretical balloon shape before pressing shown in FIG. 8B is divided into a plurality of regions along a line segment connecting one end and the other end.
[FIG. 8D] FIG. 8D is a diagram showing an example of a model in which the theoretical balloon shape before pressing shown in FIG. 8B is divided into a plurality of regions along a line segment connecting one end and the other end.
[FIG. 9] FIG. 9 is a diagram showing an example of evaluation information displayed by the display unit when the output unit D shown in FIG. 3 is a display device.

### [Embodiments for Carrying Out the Invention]

An embodiment of the present invention will be described hereunder with reference to specific examples shown in the drawings. In the drawings attached to the specification, a scale dimension, an aspect ratio and so on are changed and exaggerated from the actual ones, for the convenience of easiness in illustration and understanding. In addition, terms used herein to specify shapes, geometric conditions and their degrees, e.g., "parallel", "orthogonal", "same", etc., and values of a length and an angle are not limited to their strict definitions, but construed to include a range capable of exerting a similar function, unless otherwise specified.

In the embodiment shown below, as an example of a balloon catheter that is inserted into an organ of a subject (for example, human) and pressed against the inner surface of the organ, a balloon catheter applied to the balloon catheter treatment such as an arrhythmia of atrial fibrillation will be described. However, the balloon catheter can be used for a balloon catheter for treating other treatments such as endometriosis, cancer, etc.

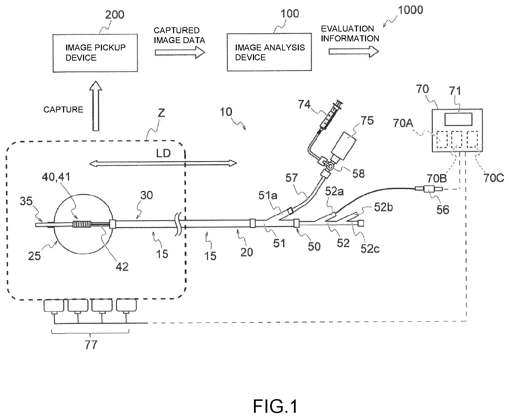

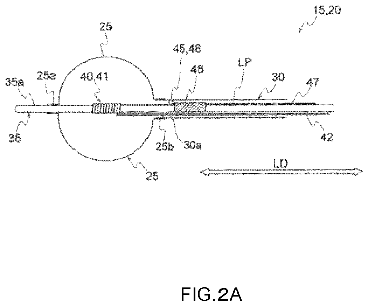

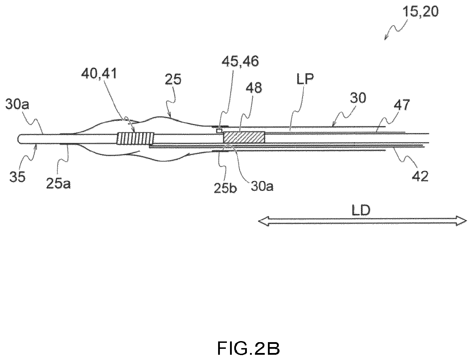

Here, FIG. 1 is a diagram showing an example of a configuration including an image analysis system comprising an image analysis device 100 according to an embodiment and a balloon catheter system 10 appli ed to balloon catheter treatment. FIG. 2A is a diagram showing an exa mple of a configuration in the vicinity of a balloon of a balloon catheter i n an inflated state. FIG. 2B is a diagram showing an example of a confi guration in the vicinity of a balloon of a balloon catheter in a deflated st ate.

First, the balloon catheter system 10 applied to the balloon catheter treatment for a subject will be described.

A balloon catheter system 10 shown in FIG. 1 has a balloon catheter 15, a control unit 70 connected to the balloon catheter 15, and an agitator 75 connected to the balloon catheter 15. The balloon catheter 15 also has a catheter body 20 having a longitudinal direction LD, and a handle 50 connected to a proximal end of the catheter body 20.

As shown in FIG. 2A, the catheter body 20 according to this embodiment has a balloon 25, an outer cylinder shaft 30 connected to a proximal end 25b of the balloon 25, an inner cylinder shaft 35 connected to a distal end 25a of the balloon 25, and a heating member 40 disposed in the balloon 25. The inner cylinder shaft 35 passes through the outer cylinder shaft 30 to extend into the balloon 25. A liquid delivery path LP in communication with the balloon 25 is formed between the outer cylinder shaft 30 and the inner cylinder shaft 35. The heating member 40 heats a liquid in the balloon 25.

The longitudinal direction LD of the catheter body 20 is specified as a direction along which center axes of the outer cylinder shaft 30 and the inner cylinder shaft 35 extending from the outer cylinder shaft 30 extend. In this specification, the term "distal" side used for respective components of the balloon catheter 15 and the catheter body 20 means a side distant from an operator (surgeon) of the handle 50 and the balloon catheter 15 along the longitudinal direction LD of the catheter body 20, in other words, a distant side. In addition, the term "proximal" side used for respective components of the balloon catheter 15 and the catheter body 20 means a side close to the operator (surgeon) of the handle 50 and the balloon catheter 15 along the longitudinal direction LD of the catheter body 20, in other words, a near side.

The balloon catheter system 10 and the balloon catheter 15 are further described in detail hereunder. First, the catheter body 20 of the balloon catheter 15 is described in detail. As described above, the catheter body 20 of the balloon catheter 15 according to this embodiment has the balloon 25, the outer cylinder shaft 30, the inner cylinder shaft 35, the heating member 40, and the temperature sensor 45.

The outer cylinder shaft 30 and the inner cylinder shaft 35 both have a tubular shape, typically a cylindrical shape. Thus, the outer cylinder shaft 30 and the inner cylinder shaft 35 respectively form lumens as inside spaces. A not-shown guide wire, for example, is inserted thorough the lumen formed by the inner cylinder shaft 35. The inner cylinder shaft 35 is inserted through the lumen formed by the outer cylinder shaft 30. Namely, the outer cylinder shaft 30 and the inner cylinder shaft 35 form a dual shaft structure. An internal diameter of the outer cylinder shaft 30 is larger than an external diameter of the inner cylinder shaft 35. Thus, a lumen remains between the outer cylinder shaft 30 and the inner cylinder shaft 35. The lumen between the outer cylinder shaft 30 and the inner cylinder shaft 35 form the liquid delivery path LP. As shown in FIG. 2A, the liquid delivery path LP is in communion with the balloon 25. The liquid delivery path LP extends up to an inside of the handle 50.

The balloon 25 is connected to the outer cylinder shaft 30 and the inner cylinder shaft 35. The balloon 25 is formed so as to be inflatable when it is filled with liquid and deflatable when a liquid is discharged therefrom. The balloon 25 is preferably shaped to fit a target site to be treated (e.g., blood vessel). As an example, the balloon 25 adapted for a pulmonary venous junction of a left atrium may have a spherical shape having a diameter between 15 mm or more and 40 mm or less. The spherical shape here includes a true spherical shape, a prolate shape, and a prolate spheroid shape. It also includes a substantially spherical shape.

In the illustrated catheter body 20, as shown in FIG. 2A and FIG. 2B, the distal end (distant end) 25a of the balloon 25 is fixed to a distal end (distant end) 35a of the inner cylinder shaft 35. The proximal end (near end) 25b of the balloon 25 is fixed to a distal end (distant end) 30a of the outer cylinder shaft 30. The balloon 25 is connected to the outer cylinder shaft 30 and the inner cylinder shaft 35 by adhesion or heat welding.

By the relative movement of the outer cylinder shaft 30 and the inner cylinder shaft 35 in the longitudinal direction LD, the balloon 25 connected to the outer cylinder shaft 30 and the inner cylinder shaft 35 deforms. In the illustrated example, the relative movement of the outer cylinder shaft 30 and the inner cylinder shaft 35 allows a dimension of the balloon 25 to be adjusted in the longitudinal direction LD. As shown in FIG. 2B, when the inner cylinder shaft 35 is relatively moved with respect to the outer cylinder shaft 30 to the distal side in the longitudinal direction LD, the balloon 25 is stretched in the longitudinal direction LD and is further strained. In the illustrated example, the movement range of the inner cylinder shaft 35 with respect to the outer cylinder shaft 30 to the distal side in the longitudinal direction is restricted by the balloon 25. When the inner cylinder shaft 35 is relatively moved with respect to the outer cylinder shaft 30 from the position shown in FIG. 2B to the proximal side in the longitudinal direction LD, the balloon 25 becomes loosened. By introducing a liquid into the loosened balloon 25, as shown in FIG. 2A, the balloon 25 can be inflated. Namely, the relative movement of the outer cylinder shaft 30 and the inner cylinder shaft 35 allows for the dimension of the balloon 25 to be adjusted in the longitudinal direction LD.

The heating member 40 is disposed in the balloon 25. The heating member 40 is a member for heating a liquid filled in the balloon 25. As an example, a nichrome wire that generates electric resistance heat can be employed as the heating member 40. A coil electrode 41 may be employed as another example of the heating member 40, as shown in Fig. 2A and FIG. 2B. By means of high-frequency current conduction (high-frequency energization) to the heating member 40 as the coil electrode 41, a high-frequency current flows between the coil electrode 41 and a counter electrode 77 (Fig. 1) disposed outside, so that the liquid positioned between the coil electrode 41 and the counter electrode 77 generates Joule heat. The counter electrode 77 is located on the back of a patient, for example.

In the example shown in FIG. 2A and FIG. 2B, the coil electrode 41 is provided on the inner cylinder shaft 35 extending inside the balloon 25. The coil electrode 41 may be made by a conductive wire wound around the inner cylinder shaft 35. The coil electrode 41 is electrically connected to a wiring 42 for high-frequency current conduction. The wiring 42 extends in the liquid delivery path LP, which serves as a lumen between the outer cylinder shaft 30 and the inner cylinder shaft 35, up to the handle 50.

The temperature sensor 45 acquires information on a liquid temperature. In this embodiment, the temperature sensor 45 has a thermosensitive part 46 disposed in the liquid delivery path LP positioned between the outer cylinder shaft 30 and the inner cylinder shaft 35.

In order to highly precisely determine a surface temperature of the balloon 25, there is a preferable length DX along the longitudinal direction LD of the outer cylinder shaft from the distal end 30a of the outer cylinder shaft 30 up to the thermosensitive part 46 of the temperature sensor 45.

As shown in FIG. 2A and FIG. 2B, the temperature sensor 45 typically has the thermosensitive part 46, and a lead wire 47 electrically connected to the thermosensitive part 46. When the temperature sensor 45 comprises a thermocouple, a part where different metals are connected serves as the thermosensitive part 46. When the temperature sensor 45 comprises a thermistor, a ceramic element serves as the thermosensitive part 46. The lead wire 47 extends inside the liquid delivery path LP, which serves as a lumen between the outer cylinder shaft 30 and the inner cylinder shaft 35, up to the handle 50.

In the illustrated example, the temperature sensor 45 is attached to the inner cylinder shaft 35. As shown in FIG. 2A and FIG. 2B, the temperature sensor 45 is attached to the inner cylinder shaft 35 by fixing the lead wire 47 of the temperature sensor 45. The thermosensitive part 46 is apart from both the outer cylinder shaft 30 and the inner cylinder shaft 35.

Next, the handle 50 is a part grasped by an operator (surgeon) during the use of the balloon catheter system 10.

The handle 50 shown in Fig. 1 has a first handle part 51 and a second handle part 52 that are slidable to each other. The first handle part (front handle part) 51 is connected to the outer cylinder shaft 30 of the catheter body 20. The second handle part (rear handle part) 52 is connected to the inner cylinder shaft 35 of the catheter body 20. By relatively moving the second handle part 52 with respect to the first handle part 51, the inner cylinder shaft 35 can be relatively moved with respect to the outer cylinder shaft 30.

As shown in Fig. 1, the handle 50 also functions as a part that connects other devices included in the balloon catheter system 10 and the balloon catheter 15.

A connector 56 extends from the second handle part 52. This connector 56 electrically connects the wiring 42 of the catheter body 20 and the lead wire 47 of the temperature sensor 45 to the external control unit 70. The connector 56 extends from one of the branches 52a provided on the second handle part 52.

The second handle part 52 has branches 52b and 52c other than the branch 52a to which the connector 56 is connected. These branches 52b and 52c function as a part through which a liquid is supplied to the lumen as an inside space of the inner cylinder shaft 35, and a part from which a guide wire inserted through the lumen of the inner cylinder shaft 35 extends. During a cardiac ablation therapy, a saline solution an amount of which is as small as about 100 ml/hour is generally injected into a body through the lumen of the inner cylinder shaft 35. The injection of saline solution effectively prevents backflow of blood into the lumen of the inner cylinder shaft 35.

In addition, as shown in Fig. 1, an extension tube 57 extends from the first handle part 51. The extension tube 57 communicates the liquid delivery path LP of the catheter body 20 with an external supply unit 74 and the agitator 75. The extension tube 57 extends from the branch 51a provided on the first handle part 51. The extension tube 57 is connected to the supply unit 74 and the agitator 75 through a valve 58. In the illustrated example, whether the supply unit 74 or the agitator 75 is communicated with the liquid delivery path LP can be selected by operating the valve 58. A three-way stopper cock may be used as the valve 58.

Next, devices constituting the balloon catheter system 10 together with the aforementioned balloon catheter 15, to be specific, the control unit 70, the supply unit 74 and the agitator 75, are described.

The illustrated control unit 70 is electrically connected to the coil electrode 41 through the wiring 42. The control unit 70 has a high-frequency current conduction controller 70A that controls high-frequency current conduction to the coil electrode 41. In the illustrated example, the high-frequency current conduction controller 70A controls the high-frequency current conduction to/through the coil electrode 41 to adjust an output from the heating member 40. The high-frequency current conduction controller 70A can control the high-frequency current conduction to the coil electrode 41 based on a surface temperature of the balloon 25 determined by a temperature computing unit 70B described later, or in accordance with a preset process, or in accordance with an input from an operator.

In addition, the control unit 70 is electrically connected to the lead wire 47 of the temperature sensor 45. The control unit 70 has the temperature computing unit 70B that computes information on a temperature acquired by the inner cylinder shaft 35. The temperature computing unit 70B calculates a liquid temperature in the liquid delivery path LP based on information on a temperature acquired by the temperature sensor 45, and further estimates a surface temperature of the balloon 25 based on the calculated liquid temperature. The temperature computing unit 70B may display a determined surface temperature of the balloon 25 on a display 71.

The control unit 70 further has an agitator controller 70C that controls the agitator 75. The agitator controller 70C may display a control condition of the agitator 75 on the display 71.

Next, the supply unit 74 supplies a liquid into the liquid delivery path LP. By supplying a liquid from the supply unit 74 to the balloon 25 through the liquid delivery path LP, the balloon 25 can be inflated as shown in FIG. 2A. On the other hand, by discharging the liquid from the supply unit 74 from the balloon 25 through the liquid delivery path LP, the balloon 25 can be deflated. The liquid to be supplied into the liquid delivery path LP may typically be a saline solution. A syringe can be used as the supply unit 74 as illustrated. However, a pump or the like can also be used as the supply unit 74.

Next, the agitator 75 is provided for agitating a liquid in the balloon 25. By agitating the liquid in the balloon 25, heat supplied into the balloon 25 can be distributed or equalized so that a surface temperature of the balloon 25 can be adjusted. The agitator 75 repeats supply of supply of liquid to the liquid delivery path LP and discharge of liquid from the liquid delivery path LP.

Next, the image analysis system 1000 that outputs evaluation information for use in balloon catheter treatment for a subject will be descri bed.

For example, as shown in FIG. 1 described above, the image analysis system 1000 includes an image pickup device 200 configured to acquire captured image data of a two-dimensional captured image, and the image analysis device 100 configured to output evaluation information for use in balloon catheter treatment for a subject.

Then, the image pickup device 200 is adapted to take an image of the balloon catheter that has been inserted into the organ of the subject (the pulmonary vein of the left atrium of the heart) and pressed against the inner surface of the organ (for example, the myocardium), according to the operation of the operator, for example. The image pickup device 200 is adapted to take an image using X-rays. That is, in the present embodiment, the captured image is a two-dimensional X-ray fluoroscopic image obtained by seeing through a balloon catheter located in the organ of the subject using X-rays.

Also, for example, as shown in FIG.1, the image analysis device 100 is adapted to output evaluation information for use in balloon catheter treatment for a subject based on the captured image data of the two-dimensional captured image acquired by the image pickup device 200, for example, depending on the operation of the operator.

The image analysis device 100 may be configured by being integrally combined with the control device 70 described above, for example.

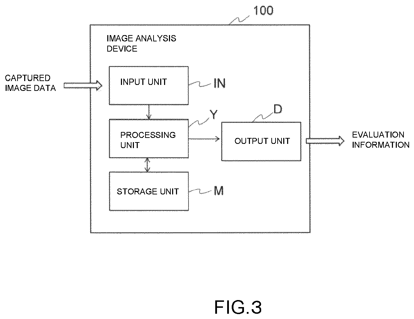

Here, FIG. 3 is a diagram showing an example of the configuration of the image analysis device 100 shown in FIG. 1. The image analysis device 100 comprises, for example, an input unit IN, a processing unit Y, an output unit D, and a storage unit M, as shown in FIG.3.

Then, the input unit IN is adapted to input the captured image data of the two-dimensional captured image obtained by imaging the balloon catheter in a state of being inserted into the organ of the subject and pressed against the inner surface of the organ.

Furthermore, the processing unit Y acquires the evaluation image data of the two-dimensional evaluation image, by executing image processing on the captured image based on the captured image data input by the input unit IN. Then, the processing unit Y acquires evaluation information regarding the state of the balloon catheter pressed against the inner surface (myocardium) of the organ of the subject, based on the acquired evaluation image data. In particular, in image processing, the processing unit Y acquires the evaluation information including the measured value obtained by measuring the shape of the balloon catheter included in the captured image or the index calculated based on the measured value.

This processing unit Y is composed of hardware such as a CPU, for example. At least a part of the processing unit Y may be configured by a software. Furthermore, in the processing unit Y, a part of the components of the processing unit Y may be able to cooperate with the above-mentioned control device 70 by communication through a network.

Furthermore, the output unit D outputs the evaluation information acquired by the processing unit Y. The output unit D is, for example, a display device that displays the evaluation information to the outside.

Furthermore, the storage unit M is designed to store captured image data, evaluation image data, and / or evaluation information. The storage unit M is, for example, a non-volatile memory such as a NAND flash memory.

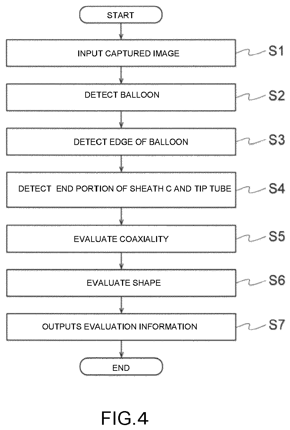

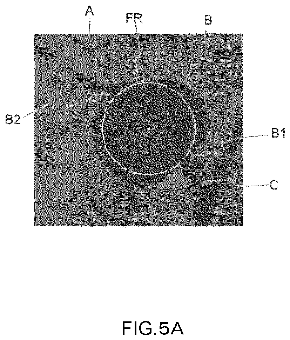

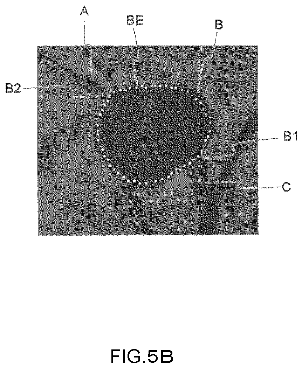

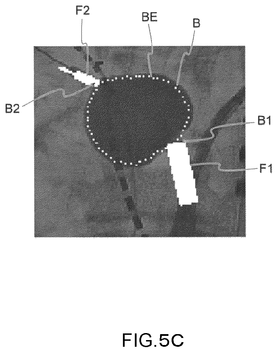

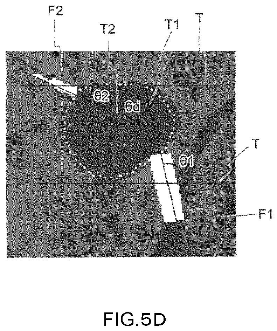

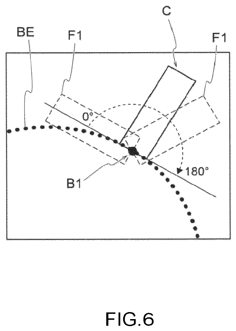

Next, a control method of the image analysis device 100 having the above configuration will be described. FIG. 4 is a diagram showing an example of a control method of the image analysis device 100 shown in FIG.3. FIG. 5A is a diagram showing an example of image processing that executes a Sobel filter process and a Ring filter process for detecting a balloon in a captured image. FIG. 5B is a diagram showing an example of image processing for executing a process of detecting the edge of a balloon by performing a shading calculation on radiation from the center point of the balloon detected by the process of FIG. 5A. FIG. 5C is a diagram showing an example of image processing for executing a rectangular detection filter process for detecting an end portion and a tip tube of a sheath in a captured image. FIG. 5D is a diagram showing an example of image processing for calculating the angle difference between the end portion of the sheath and the tip tube in a captured image. FIG. 6 is a diagram showing an example of a model of rectangular detection filtering processing shown in FIG. 5C.

First, the image pickup device 200 acquires the captured image data of the two-dimensional captured image, by imaging the balloon catheter that is inserted into the organ of the subject during the procedure and pressed against the inner surface (the myocardium) of the organ, for example, in response to the operation of the operator.

Then, for example, as shown in Fig. 4, the input unit IN of the image analysis device 100 inputs the captured image data of the two-dimensional captured image, output from the image pickup device 200, obtained by imaging the balloon catheter which is inserted into the organ of the subject during the procedure and pressed against the inner surface (the myocardium) of the organ (the step S1 in FIG. 4).

Next, in the image processing, the processing unit Y executes noise reduction processing, shading detection processing, or pattern matching processing on the captured image input by the input unit IN.

Then, in the image processing, the processing unit Y detects the balloon B of the balloon catheter from the captured image (the step S2 in FIG. 4).

Here, in the image-processed captured image, for example, as shown in FIG. 5A. it is assumed that the balloon catheter includes: the tip tube A; the sheath C (corresponding to the outer cylinder shaft 30 shown in FIGS. 1 and 2A and B2 described above); the catheter tube housed in the sheath C (corresponding to the inner cylinder shaft 35 shown in FIGS. 1 and 2A and 2B described above); the balloon B having one end (the proximal end 25b of the balloon 25 shown in FIG. 2B described above) B1 connected to the end portion of the sheath C (corresponding to the balloon 25 shown in FIGS. 1 and 2A and 2B described above); and the tip tube A (corresponds to the distal end (tip) 35a of the inner cylinder shaft 35) which is connected to the other end B2 (corresponding to the distal end 25a of the balloon 25 in FIG. 2B) of the balloon B, is continuous with the catheter tube, and leads the sheath C. In this case, for example, as shown in FIG. 5A, in the image processing, the processing unit Y detects the balloon B from the captured image by executing the Sobel filter processing and the Ring filter processing on the captured image. Here, the processing unit Y may detect the balloon B by executing only one of the Sobel filter processing and the Ring filter processing on the captured image.

Next, in the image processing, the processing unit Y acquires the shape of the balloon B in the captured image by detecting the edge of the balloon B of the balloon catheter (the step S3 in FIG. 4). Here, for example, as shown in FIG. 5B, the shading calculation is performed radially (for example, 2 ° interval) from the center of the balloon B detected by the process of FIG. 5A, the luminance change point is measured, and the edge BE is plotted.

Next, in image processing, the processing unit Y detects the end portion of the sheath C and the tip tube A (the step S4 in FIG. 4). For example, as shown in FIG. 5C, in image processing, the processing unit Y detects the end portion of the sheath C and the tip tube A by the first and second rectangular detection filters F1 and F2, from the vicinity of one end B1 or the other end B2 (edge BE) of the balloon B detected from the captured image. More specifically, for example, as shown in FIG. 6, the processing unit Y detects the one having the closest shape to the rectangular detection filter as the end portion of the sheath C or the tip tube A, by rotating the first and second rectangular detection filters F1 and F2 by 0 ° to 180 ° (for example, 2 ° interval) around the detected edge BE (including one end B1 and the other end B2) of the balloon B.

In image processing, for example, the size of the first rectangular detection filter F1 for detecting the end of the sheath C is set to be different from the size of the second rectangular detection filter F2 for detecting the tip tube A, and the sizes of the first and second rectangular detection filters F1 and F2 are set so as not to interfere with other cables, wires, or the like.

Furthermore, in image processing, the wires and the like constituting the balloon catheter may be detected in order to evaluate the coaxiality, but the wires and the like may not be distinguishable from other wirings and the like. Therefore, by targeting the sheath C and the tip tube A, which are unique configurations of the balloon catheter, with the first and second rectangular detection filters F1 and F2, the sheath C and the tip tube A can be detected more accurately.

In this way, the processing unit Y detects the balloon B located in the organ of the subject, the end of the sheath C (or the end of the catheter tube) in contact with one end B1 of the balloon B, and the tip tube A in contact with the other end B2 of the balloon B, from the captured image (FIGS. 5C and 6).

Next, in image processing, the processing unit Y evaluates the coaxiality of the balloon catheter in the pressed state (the step S5 in FIG. 4). For example, as shown in FIG. 5D, the processing unit Y acquires (calculates) the coaxiality indicating the relationship between the first axial direction T1, which is the direction of the end of the sheath C or the central axis of the catheter tube, and the second axial direction T2, which is the direction of the central axis of the tip tube A, detected from the image-processed captured image, as evaluation information.

Specifically, in image processing, for example, as shown in FIG. 5D, the processing unit Y calculates a first angle θ1 of the first axial direction (the long side direction of the end of the rectangular sheath C) T1 of the end portion of the sheath C with respect to the reference direction T and a second angle θ2 of the second axial direction (the long side direction of rectangular tip tube A) T2 of the tip tube A with respect to the reference direction T, in the two-dimensional captured image. Then, the processing unit Y acquires (calculates) the angle difference θd (the coaxiality), which is the difference between the first angle θ1 and the second angle θ2, as the evaluation information of the coaxiality.

For example, the processing unit Y evaluates a state in which the end portion of the sheath C and the tip tube A are aligned with the balloon B (there is little difference in angle between the end of the sheath C and the tip tube A) as having high coaxiality.

Further, the processing unit Y may evaluate the change in the coaxiality of the balloon catheter within a predetermined period during the balloon catheter treatment, by performing the analysis of the moving image based on the captured image input from the input unit IN during the balloon catheter treatment.

Next, in image processing, the processing unit Y evaluates the shape of the balloon catheter in the pressed state (the step S6 in FIG. 4). The processing unit Y may evaluate the change in the shape of the balloon B of the balloon catheter within a predetermined period during the treatment of the balloon catheter, by performing the analysis of the moving image based on the captured image input from the input unit IN during the balloon catheter treatment.

Next, the output unit D outputs the evaluation information acquired by the processing unit Y (the step S7 in FIG. 4). Here, the output unit D displays, for example, the character information of the shape variation and the coaxiality, and the evaluation image including notations of the shape of the balloon B shown in FIG. 5D, the reference direction T, the first angle θ1, the first axial direction T1, the second angle θ2, the second axial direction T2, and the angle difference θd, as the evaluation information.

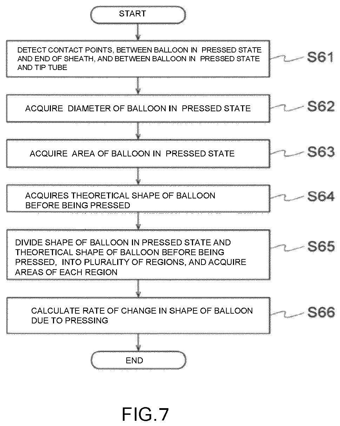

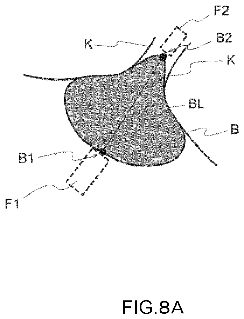

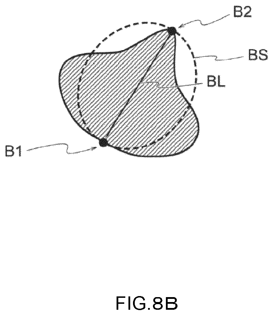

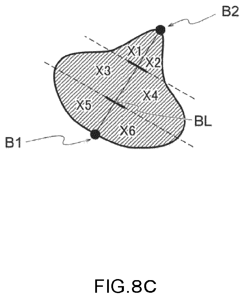

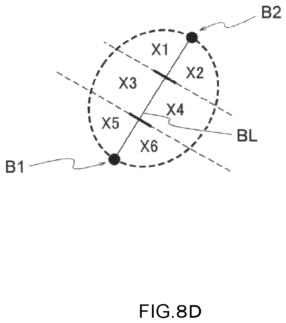

Here, FIG. 7 is a diagram showing a detailed example of a step of evaluating a shape in the control flow shown in FIG. 4. FIG. 8A is a diagram showing an example of a two-dimensional image of a balloon catheter in a state of being pressed against the inner surface (the myocardium) of an organ of a subject, which is acquired in image processing. FIG. 8B is a diagram showing an example in which a two-dimensional image of the balloon shown in FIG. 8A and the theoretical outer shape of the balloon before pressing are superimposed. FIG. 8C is a diagram showing an example of a model in which the theoretical balloon shape before pressing shown in FIG. 8B is divided into a plurality of regions along a line segment connecting one end and the other end. FIG. 8D is a diagram showing an example of a model in which the theoretical balloon shape before pressing shown in FIG. 8B is divided into a plurality of regions along a line segment connecting one end and the other end.

In the step S6 for evaluating the shape of the balloon B in the state of being inserted into the organ of the subject and pressed against the inner surface K of the organ in the control flow shown in FIG. 4 described above, in image processing, the processing unit Y detects the first contact point (ie, one end of the balloon B) B1 between the detected edge of balloon B and the end of sheath C, and the second contact point (that is, the other end of the balloon B) B2 between the detected edge of the balloon B and the tip tube A (the step S61 in FIG. 7, FIG. 8A).

Next, the processing unit Y calculates the length of the line segment BL connecting the detected first contact point B1 and the second contact point B2, as the diameter value (the diameter (when the shape of balloon B in the captured image is circular), as the major axis (when the shape of balloon B in the captured image is elliptical), or as the minor axis (when the shape of balloon B in the captured image is elliptical)) of the balloon B in a free state (before being pressed) that is not pressed against the inner surface of the organ before the procedure (the step S62 in FIG. 7).

Next, the processing unit Y calculates the first balloon area of the first shape of the balloon B whose edge is detected in the pressed state in which the balloon B is pressed against the inner surface of the organ during the procedure (the step S63 in FIG. 7).

Next, the processing unit Y acquires (calculates) the theoretical second shape, which is the circle or ellipse, of the balloon B in a free state (before being pressed) that is not pressed against the inner surface of the organ before the procedure, based on the calculated diameter, major axis, or minor axis of balloon B, and the first balloon area (the step S64 in FIG. 7, FIG. 8B).

Next, the processing unit Y divides the first shape of the balloon B in the pressed state into a plurality of regions X1 to X6, and calculates the area of each region X1 to X6 of the divided first shape (the step S65 in FIG. 7, and FIG. 8C). For example, the processing unit Y divides the first shape of the balloon B in the pressed state into a plurality of regions along the line segment BL. Furthermore, the processing unit Y divides the first shape of the balloon B in the pressed state into a plurality of regions around the line segment BL. In particular, in this embodiment, for example, as shown in FIG. 8C, the processing unit Y divides the first shape into sections of each 1/3 of the length of the line segment BL along the line segment BL, and divides the first shape around the line segment BL, so that the processing unit Y divides the first shape of the balloon B in the pressed state into six regions X1 to X6.

Furthermore, the processing unit Y divides the theoretical second shape of the balloon B into a plurality of regions X1 to X6 in the same manner as in dividing the first shape, and calculates the area of each region X1 to X6 of the divided second shape (the step S65, FIG. 8D). For example, the processing unit Y divides the theoretical second shape of the balloon B into a plurality of regions along the line segment BL. Furthermore, the processing unit Y divides the theoretical second shape of the balloon B into a plurality of regions around the line segment BL. In particular, in the present embodiment, the theoretical second shape of the balloon B is divided into six regions X1 to X6. For example, as shown in FIG. 8D, the processing unit Y divides the second shape into sections of every 1/3 of the length of the line segment BL along the line segment BL, and divides the second shape around the line segment BL.

Here, in this step S65, for example, in image processing, the processing unit Y acquires the number of first pixels, which is the total number of pixels of the captured image corresponding to each region X1 to X6 of the detected first shape of the balloon B. Furthermore, the processing unit Y acquires the number of second pixels, which is the total number of pixels of the captured image corresponding to each region X1 to X6 of theoretical second shape of a free-state balloon B that is not pressed against the inner surface of the pre-procedural organ (before being pressed). By acquiring the number of the first and second pixels, the area corresponding to each region X1 to X6 can be acquired.

Next, the processing unit Y calculates the rate of change in the shape of the balloon B due to pressing against the inner surface of the organ (the step S66), by comparing the area of each region X1 to X6 of the first shape of the balloon B (FIG. 8C) and the area of each region X1 to X6 of the second shape of the balloon B (FIG. 8D), for each area X1 to X6 corresponding to the position in the captured image.

In particular, in this step S66, the processing unit Y can evaluate the shape (obtain the rate of change in the shape) of the balloon B pressed against the inner surface of the organ during the procedure, for example, based on the difference between the number of first pixels (that is, the area) and the number of the second pixels (that is, the area) in each of the regions X1 to X6 acquired in the step S65 described above.

In the calculation of the above area, it is assumed that the length (the diameter of the balloon B) of the line segment BL connecting the first contact point B1 and the second contact point B2 has not changed, by pressing the balloon B against the inner surface of the organ.

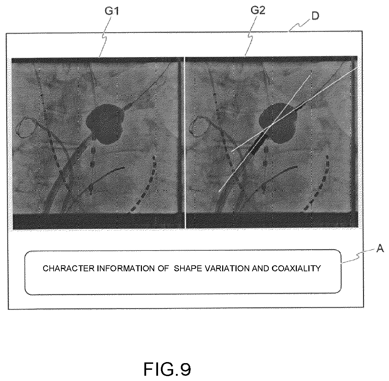

Here, FIG. 9 is a diagram showing an example of evaluation information displayed by the display unit D when the output unit D shown in FIG. 2 is a display device.

For example, as shown in Fig. 9, the output unit (the display device) D may further display the captured image G1 together with the evaluation image G2 obtained by the image processing and the character information A regarding the rate of change in shape and the coaxiality.

In the example shown in Fig. 9, the notation of the reference direction T, the first angle θ1, the first axial direction T1, the second angle θ2, the second axial direction T2, and the angle difference θd described above is omitted. However, for example, as shown in FIG. 5D, the notation of the reference direction T, the first angle θ1, the first axial direction T1, the second angle θ2, the second axial direction T2, and the angle difference θd may be displayed.

As described above, the image analysis device 100 according to the present embodiment can output evaluation information for use in balloon catheter treatment for a subject in real time during the procedure using the balloon catheter.

Thereby, for example, the doctor who is the operator can improve the success rate of the treatment in the balloon catheter treatment for the subject (human), by properly determining whether the surface temperature of the balloon is correctly transmitted to the myocardium, using the evaluation information output in real time from the image analysis device 100, the contact state between the balloon and the myocardium is recognized, during the procedure with the balloon catheter.

While certain embodiments have been described, these embodiments have been presented by way of example only, and are not intended to limit the scope of the inventions. The embodiments may be embodied in a variety of other forms. Furthermore, various omissions, substitutions and changes in the form of the methods and systems described herein may be made without departing from the spirit of the inventions. The embodiments and their modifications are included in the scope and the subject matter of the invention, and at the same time included in the scope of the claimed inventions and their equivalents.

### [Industrial applicability]

The present invention can be used for a balloon catheter system and a balloon catheter for treating arrhythmia such as atrial fibrillation, endometriosis, cancer and the like.

### [Explanation of References]

- 100: image analysis device
- IN: input unit
- Y: processing unit
- D: output unit
- M: storage unit

## Claims

1. An image analysis device that outputs evaluation information for use in a balloon catheter treatment for a subject, the image analysis dev ice comprising:
an input unit configured to input captured image data of a two-di mensional captured image of a balloon catheter inserted into an organ of the subject and pressed against an inner surface of the organ;
a processing unit configured to obtain evaluation image data of a two-dimensional evaluation image, by executing an image processing on the captured image based on the captured image data input by the input unit, and configured to obtain evaluation information regarding a state o f the balloon catheter pressed against the inner surface of the organ bas ed on the evaluation image data; and
an output unit configured to output the evaluation information ac quired by the processing unit.

2. The image analysis device according to claim 1,
wherein, in the image processing,
the processing unit acquires the evaluation information including t he measured value obtained by measuring the shape of the balloon cath eter included in the captured image or an index calculated based on the measured value, and
the output unit outputs the evaluation information acquired by th e processing unit.

3. The image analysis device according to claim 1,
wherein the captured image is a two-dimensional X-ray fluoroscop ic image taken by seeing through the balloon catheter located in the org an of the subject, using X-rays.

4. The image analysis device according to claim 1,
wherein the balloon catheter comprises:
a sheath;
a catheter tube housed in the sheath;
a balloon having one end connected to an end of the sheath; and
a tip tube connected to the other end of the balloon and configur ed to lead the sheath.

5. The image analysis device according to claim 4,
wherein, in the image processing, the processing unit detects the balloon located in the organ of the subject, the end of the sheath in con tact with one end of the balloon or the end of the catheter tube, and tip tube in contact with the other end of the balloon, from the captured im age, by executing noise reduction processing, shading detection processin g, or pattern matching processing on the captured image input by the in put unit.

6. The image analysis device according to claim 5,
wherein, in the image processing,
the processing unit detects the end portion of the sheath and the tip tube by a rectangular detection filter from a vicinity of the edge of t he balloon detected from the captured image.

7. The image analysis device according to claim 6,
wherein, in the image processing,
a size of the first rectangular detection filter for detecting the end of the sheath is different from a size of the second rectangular detectio n filter for detecting the tip tube, and the size of the first rectangular de tection filter and the size of the second rectangular detection filter are se t to sizes that do not interfere with a wire.

8. The image analysis device according to any one of claims 5 to 7,
wherein, in the image processing,
the processing unit detects a coaxiality indicating a relationship b etween a first axial direction, of the end of the sheath or the catheter tu be, and a second axial direction, of the tip tube, from the image-process ed captured image, and the processing unit acquires the detected coaxial ity as the evaluation information.

9. The image analysis device according to claim 8,
wherein, in the image processing, the processing unit calculates a first angle in the first axial direction of the end of the sheath with resp ect to the reference direction and a second angle in the second axial dir ection of the tip tube with respect to the reference direction, in the two-dimensional captured image, and the processing unit obtains an angle dif ference, which is the difference between the first angle and the second a ngle, as the evaluation information of the coaxiality.

10. The image analysis device according to any one of claims 5 to 9,
wherein, in the image processing, the processing unit obtains nu mber of first pixels, which is total number of pixels of the captured imag e corresponding to a detected first shape of the balloon, and obtains nu mber of second pixels, which is total number of pixels of the captured i mage corresponding to a theoretical second shape of the balloon before being pressed against the inner surface of the organ, and the processing unit evaluates the shape of the balloon pressed against the inner surface of the organ based on a difference between the number of first pixels a nd the number of second pixels.

11. The image analysis device according to claim 10,
wherein, in the image processing,
the processing unit detects a first contact point between the dete cted edge of the balloon and the end of the sheath, and a second conta ct point between the detected edge of the balloon and the tip tube,
the processing unit calculates a length of the line segment connec ting the first contact point and the second contact point detected, as a d iameter of the balloon before being pressed against the inner surface of the organ,
the processing unit calculates a first balloon area of the first shap e of the balloon whose edge is detected in a pressed state pressed again st the inner surface of the organ,
the processing unit obtains a theoretical second shape of a circle or ellipse of the balloon before the balloon is pressed against the inner s urface of the organ, based on the calculated diameter of the balloon and the first balloon area,
the processing unit divides the first shape of the balloon in the pr essed state into a plurality of regions,
the processing unit calculates area of each of the regions of the d ivided first shape,
the processing unit divides the theoretical second shape of the ba lloon into a plurality of regions in the same manner as the dividing of th e first shape,
the processing unit calculates the area of each region of the seco nd shape divided, and
the processing unit calculates a rate of change in the shape of th e balloon due to pressing against the inner surface of the organ, by com paring the area of each region of the first shape of the balloon and the area of each region of the second shape of the balloon for each region c orresponding to a position in the captured image.

12. The image analysis device according to claim 11,
wherein, in the image processing,
the processing unit divides the first shape of the balloon in the pr essed state into the plurality of regions along the line segment, and
the processing unit divides the theoretical second shape of the ba lloon into the plurality of regions along the line segment.

13. The image analysis device according to claim 12,
wherein, in the image processing, the processing unit divides the first shape of the balloon in the pressed state into a plurality of regions around the line segment, and divides the theoretical second shape of the balloon into a plurality of regions around the line segment.

14. The image analysis device according to claim 13,
wherein, in the image processing,
the processing unit divides the first shape of the balloon in the pr essed state into six regions, by dividing along the line segment into secti ons every 1/3 of the length of the line segment and dividing by the cent er of the line segment, and
the processing unit divides the theoretical second shape of the ba lloon into six regions, by dividing along the line segment into sections ev ery 1/3 of the length of the line segment and dividing by the center of t he line segment.

15. An image analysis system that outputs evaluation information for use in balloon catheter treatment for subjects, the image analysis system comprises:
an image pickup device configured to obtain captured image data of a two-dimensional captured image, by imaging a balloon catheter inse rted into the organ of the subject and pressed against the inner surface of the organ; and
an image analysis device configured to output evaluation informati on for use in balloon catheter treatment for the subject based on the ca ptured image data of the captured image,
wherein the image analysis device comprises:
an input unit configured to input captured image data of a two-di mensional captured image obtained by the image pickup device;
a processing unit configured to obtain evaluation image data of a two-dimensional evaluation image, by executing an image processing on the captured image based on the captured image data input by the input unit, and configured to obtain evaluation information regarding a state o f the balloon catheter pressed against the inner surface of the organ bas ed on the evaluation image data; and
an output unit configured to output the evaluation information ac quired by the processing unit.
